Europäisches Patentamt

European Patent Office

Office européen des brevets

(19)

(11) Veröffentlichungsnummer: **0 191 113 B1**

(12) **EUROPÄISCHE PATENTSCHRIFT**

(45) Veröffentlichungstag der Patentschrift:
31.01.90

(21) Anmeldenummer: 85100195.8

(22) Anmeldetag: 10.01.85

(51) Int. Cl.⁴: **A 61 M 5/32**

(54) Vorrichtung für Injektionsspritzen zur intramuskulären und subkutanen Injektion.

(43) Veröffentlichungstag der Anmeldung:
20.08.86 Patentblatt 86/34

(45) Bekanntmachung des Hinweises auf die Patenterteilung:
31.01.90 Patentblatt 90/5

(84) Benannte Vertragsstaaten:
AT CH DE FR GB IT LI NL SE

(56) Entgegenhaltungen:
DE-B- 1 166 419
FR-A- 2 315 284
FR-A- 2 408 354
US-A- 2 393 143
US-A- 3 356 089

(73) Patentinhaber: VEGA Grieshaber GmbH & Co.,
D-7620 Wolfach (DE)

(72) Erfinder: Grieshaber, Bruno, Allgeirhof, D-7620 Wolfach
(DE)

(74) Vertreter: Strasse, Joachim, Dipl.-Ing., Strasse und
Stoffregen European Patent Attorneys
Zweibrückenstrasse 17, D-8000 München 2 (DE)

**Beschreibung**

Eine Vorrichtung für Injektionsspritzen zur intramuskulären und subkutanen Injektion in der Human- und der Veterinärmedizin mit einem die Injektionsnadel der Injektionsspritze zumindest auf einem Teil ihrer Länge konzentrisch umgebenen Führungskörper, der an seinem einen, zum Aufsetzen auf die Haut vorgesehenen Ende eine Öffnung hat, wobei die Injektionsnadel in einer ersten Endlage, in der keine Kraft auf die Injektionsspritze ausgeübt wird, mit ihrer Spitze nicht über die Öffnung hinausragt und in einer zweiten Endlage durch Krafteinwirkung auf die Injektionsspritze mit ihrer Spitze um eine Strecke, deren Länge von der Lage eines Anschlags im Führungskörper und von einem gegen den Anschlag angelehnten Vorsprung abhängt, über das Ende des Führungskörpers hinausragt.

Eine derartige Vorrichtung für Injektionsspritzen ist bereits bekannt (DE-A 2 929 425). Bei dieser bekannten Vorrichtung ist der Führungskörper als rohrförmige Hülse ausgebildet, deren Innendurchmesser etwas größer als der Außendurchmesser der zugehörigen Spritze ist. In der Hülse befindet sich ein erster ringförmiger Vorsprung, dessen Durchmesser in Richtung des auf die Haut aufsetzbaren Endes der Hülse stetig zunimmt. Der erste Vorsprung dient als Klemmelement für die der Injektionsnadel zugewandte Stirnseite der Injektionsspritze. Ein zweiter ringförmiger Vorsprung ist im Abstand vom ersten Vorsprung nahe an dem auf die Haut setzbaren Ende der Hülse angeordnet. Der zweite Vorsprung bildet einen Anschlag für die Stirnseite der Injektionsspritze. Der Abstand dieses Anschlags von der Öffnung der Hülse ist so auf den Abstand der Spitze der Injektionsnadel von der Stirnseite der Injektionsspritze abgestimmt, daß die Spitze der Injektionsnadel in der zweiten Endlage, in der die Stirnseite am Anschlag anliegt, um ein bestimmtes Stück über die Hülse hinausragt. Wenn die Hülse mit ihrem Ende gegen die Haut angedrückt ist, befindet sich die Spitze der Injektionsnadel um dieses Stück innerhalb des Körpers.

Bekannt ist auch eine Injektionsvorrichtung, bei der eine handelsübliche Injektionsspritze mit aufgesetzter handelsüblicher Injektionsnadel in einer Hülse gehalten ist, die in einer äußeren Hülse gegen den Druck einer Schraubenfeder teleskopartig verschiebbar ist. Bei gespannter Schraubenfeder sind die beiden Hülsen gegenseitig mit einem Rasthebel verriegelt. Nach dem Lösen der Verriegelung schnellt die Injektionsnadel unter der Federkraft um ein Stück aus der Öffnung der Hülse heraus (DE-U 1 983 641).

Weiterhin ist ein Zusatzgerät für eine Injektionsspritze bekannt, das eine federnde Hülse als Halter für den zylindrischen Teil der Injektionsspritze und eine Kammer enthält, in die ein Schaft hineinragt, an dessen äußerem Ende eine Gabel als Anschlag für die eine Stirnseite des Zylinders der Injektionsspritze befestigt ist. Der Schaft ist von einer Spiralfeder umgeben, unter deren Federvorspannung der Schaft in die Kammer hineingezogen wird. Der Schaft läßt sich entgegen der Kraft der Feder ein Stück aus der Kammer herausziehen und in dieser Lage arretieren. Bei herausgezogenem, arretiertem Schaft wird die Injektionsspritze in den Halter eingesetzt und axial so weit verschoben, bis die der Injektionsnadel abgewandte Stirnseite des Spritzenzylinders an der Gabel anliegt. Nach dem Lösen der Arretierung drückt die Feder über den Schaft und die Gabel die Injektionsspritze aus der Hülse ein Stück heraus. Bei auf die Haut aufgesetzter Hülse erfolgt hierbei der Einstich (DE-A 1 766 310).

Schließlich ist eine Injektionsspritze bekannt, die eine von einer Einstechvorrichtung umgebene Injektionsnadel enthält. Die Einstechvorrichtung weist eine mit einem zentrischen Kanal versehene Einstechspritze auf, die über einen federnden Bügel mit dem Fuß der Injektionsnadel verbunden ist. Der Bügel, der unter einer Federspannung das Bestreben hat, sich mit seinen beiden Abschnitten zusammenzufalten, wird durch Fingerdruck gesteuert. Wenn die beiden Abschnitte des Bügels längs einer Geraden ausgerichtet sind, wird die Einstechspritze in die Haut eingeführt. Danach wird von Hand der Druck auf die Knickstelle zwischen den beiden Bügelabschnitten allmählich vermindert, wobei die Spitze der Injektionsnadel tiefer in das Gewebe eindringt (US-A 3 840 008).

Außerdem ist aus der FR-A 2 315 284 ein Führungskörper für eine Injektionsspritze mit einem an den Körper des Patienten anzulegenden Ende bekannt, in dem eine Injektionsnadel zwischen einer eingezogenen Lage, und einer Lage, in der sie um einen bestimmten Betrag aus dem Führungskörper hervorsteht, selbsttätig verschiebbar ist. Hierbei besitzt der Führungskörper ebenfalls einen um 90° seitlich abstehenden Haltegriff. Weiterhin ist hier auch ein Anschlag vorgegeben, damit die Nadel nicht zuerst ausgefahren wird.

Bei den oben beschriebenen Vorrichtungen bleibt nach dem Einstich der Injektionsnadel die Verbindung zwischen dem Führungskörper und der Injektionsspritze bestehen. Die Einstichtiefe kann daher nicht über dasjenige Maß erhöht werden, das durch die Anordnung des Anschlags im Führungskörper, den Abstand des Anschlags vom Ende des Führungskörpers und die Länge der Strecke zwischen der Spitze der Injektionsnadel und dem Anschlag vorgegeben ist. Es kann jedoch auch notwendig werden, die Injektionsnadel nach dem Einstich auf eine vorgegebene Tiefe anschließend noch tiefer in das Gewebe oder in Körperhohlräume einzuführen. Der Erfindung liegt daher die Aufgabe zugrunde, eine Vorrichtung zu entwickeln, die zunächst einen Einstich auf eine vorgegebene Länge und danach ein tieferes Einführen der Injektionsnadelspitze erlaubt.

Diese Aufgabe wird erfindungsgemäß durch die im Anspruch 1 beschriebenen Maßnahmen gelöst. Die beiden Hälften des Führungskörpers lassen sich bei der im Anspruch 1 angegebenen Vorrichtung nach dem Einstechen der Injektionsnadel voneinander entfernen und beiseite legen. Um das vom Führungskörper umgebene Stück der Injektionsnadel ist nach der Entfernung des Führungskörpers die Injektionsnadel tiefer in das Gewebe bzw. in Körperhohlräume einführbar.

Vorzugsweise sind die beiden Hälften des Führungskörpers je an einem Ende von Griffstücken einer Zange befestigt, deren Griffstücke je an einem Ende aneinander befestigt und durch Überwinden der aneinanderbewegenden oder auseinanderspreizenden Federkraft zu betätigen sind. Die Zange ist zweckmäßigerweise nach Art einer Pinzette ausgebildet, die flache je mit den Führungskörpern verbundene Vorderenden enthält. Es ist auch günstig, als Zange einen federnden U-förmig gebogenen Bügel zu verwenden. Die beiden Griffstücke der Zange spreizen aufgrund der federnden Ausbildung die Führungskörperhälften auseinander. Vor dem Einstich werden die Führungskörperhälften durch Druck auf die Griffstücke aneinandergedrückt, wobei sich die Injektionsnadel im Kanal befindet. Danach erfolgt der Einstich auf die vorgegebene Tiefe. Anschließend wird der Druck auf die Griffstücke vermindert, bis die beiden Führungskörperhälften auseinandergespreizt sind. Sodann kann die aus den Führungskörperhälften und der Zange bestehende Einheit beiseite gelegt werden.

Es ist auch möglich, die Zange so auszubilden, daß die Federkraft die Führungskörperhälfte nicht auseinanderspreizt, sondern in Schließrichtung beansprucht. Die beiden Führungskörperhälften werden im letzten Fall durch manuell auf die Griffstücke ausgeübtem Druck auseinandergespreizt. Die Zange kann hierfür zwei Backen mit Griffstücken enthalten, die um ein Gelenk schwenkbar oder mit gekreuzten Griffen versehen sind. Die letzteren Ausführungsformen haben den Vorteil, daß die Führungskörperhälften die Injektionsnadel auch ohne manuelle Kraftausübung umschließen. Der Führungskörper muß daher beim Einstich nicht mit der Hand gehalten werden. Vorteilhaft ist bei den oben beschriebenen Vorrichtungen ferner, daß die Spitze der Nadel auf die vorgesehene Einstichstelle gesetzt werden kann, bevor die Führungskörperhälften um die Injektionsnadel angeordnet werden. Die Einstichstelle läßt sich daher exakter auswählen. Die Führungskörper bestehen vorzugsweise aus einem physiologisch inaktiven Kunststoff, insbesondere Polytetrafluoräthylen oder Polyäthylen.

Weitere Einzelheiten, Merkmale und Vorteile der Erfindung ergeben sich aus der folgenden Beschreibung von zeichnerisch dargestellten Ausführungsbeispielen.

Es zeigen:

Fig. 1 eine Ausführungsform eines Führungskörpers, der zangenartig ausgebildet ist, in perspektivischer Ansicht;

Fig. 2 eine perspektivische Ansicht einer zweiten Ausführungsform eines zangenartig ausgebildeten Führungskörpers;

Fig. 3 eine perspektivische Ansicht einer dritten Ausführungsform eines zangenartig ausgebildeten Führungskörpers;

Fig. 4 eine Ansicht von oben einer weiteren Ausführungsform eines zangenartig ausgebildeten Führungskörpers und

Fig. 5 eine perspektivische Ansicht einer zangenartigen weiteren Ausführung mit gekreuzten Griffen.

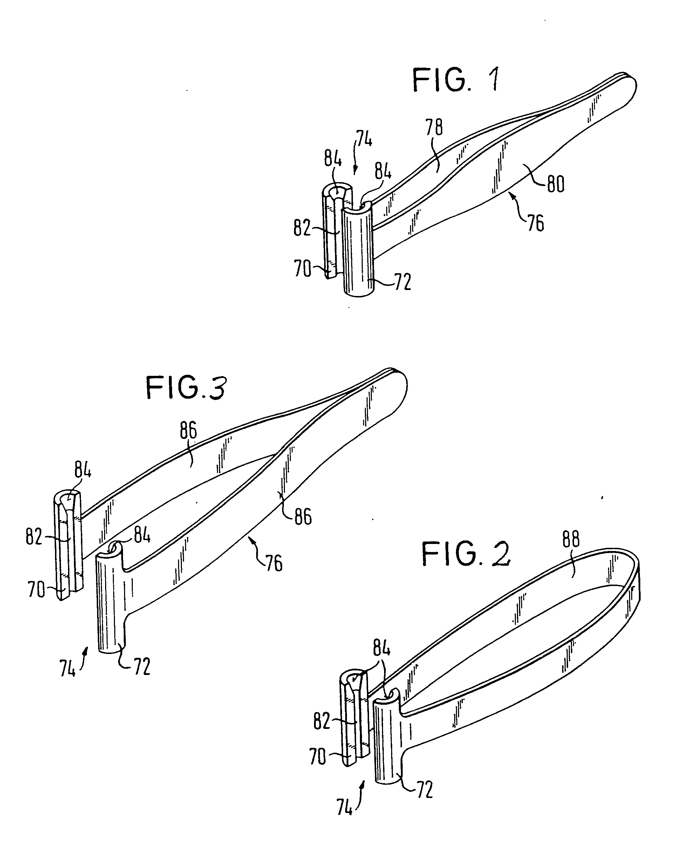

Die Figur 1 zeigt einen zwei Hälften 70, 72 aufweisenden Führungskörper 74. Die Hälften 70, 72 sind symmetrisch zueinander und entsprechen den durch mittige Längsteilung eines rotationssymmetrischen Führungskörpers entstandenen Hälften. Die beiden Hälften 70, 72 sind mit den Enden eines Trägers 76 verbunden, der ein Aneinanderlegen und ein Auseinanderspreizen beider Hälften 70, 72 zuläßt. Der Träger 76 ist bei der in Figur 1 dargestellten Ausführungsform pinzettenartig ausgebildet und enthält zwei federnd nebeneinander angeordnete Griffstücke 78, 80, die an ihren den Hälften 70, 72 entgegengesetzten Enden miteinander verbunden sind. Unter dem Einfluß der Federwirkung der Griffstücke 78, 80 sind die Hälften 78, 80 auseinandergespreizt. Die beiden Hälften 70, 72, die ebenso wie die Griffstücke 78, 80 zweckmäßigerweise aus Metall oder Kunststoff bestehen, weisen jeweils Kanäle 82 und Ausnehmungen 84 auf.

Die in Figur 3 dargestellte Ausführungsform unterscheidet sich von der Vorrichtung gemäß Figur 1 durch einen andersartigen Träger 76. Der Träger 76 gemäß Figur 3 hat zwei auf ihrer gesamten Länge gleich breite Griffstücke 86. Die Figur 6 zeigt eine Ausführungsform mit einem Träger 88, der als U-förmig gebogener Bügel ausgebildet ist, dessen Enden mit den Hälften 70, 72 verbunden sind. Auch bei den in Figuren 2 und 3 dargestellten Vorrichtungen bewirkt die federnde Ausbildung des Bügels 88 bzw. der Griffstücke 86 ein Auseinanderspreizen der beiden Hälften 70, 72 des Führungskörpers 74.

Eine Injektionsspritze mit aufgesteckter Injektionsnadel wird zunächst ohne eine der in den Figuren 1, 2 und 3 dargestellten Vorrichtungen von Hand auf diejenige Stelle der Haut ausgerichtet, an der ein Einstich erfolgen soll. Die Spitze wird dabei nur schwach gegen die vorgesehene Einstichstelle gedrückt. Anschließend wird eine der Vorrichtung gemäß Figuren 1 bis 3 auf den Nadelabschnitt derart ausgerichtet, daß sich dieser in der Mitte zwischen den Kanälen 82 befindet. Danach werden durch manuellen Druck auf die Griffstücke 78, 80 bzw. 86 oder auf den Bügel 88 die beiden Hälften 70, 72 aneinandergelegt. Die Hälften 70, 72 umgeben dann den Nadelabschnitt. Sodann wird durch einen z.B. schlagartig aufgebrachten Druck auf das freie Ende der Injektionsspritze eine axiale Verschiebung der Injektionsspritze nebst Injektionsnadel bewirkt, die in den Kanälen 82 gleitet, bis die Injektionsnadelhalterung mit ihrem an den Nadelabschnitt angrenzenden Teil in die Ausnehmung 84 eingefügt ist. In dieser Stellung ist die Spitze der Injektionsnadel um ein vorgegebenes Stück in den Körper eingedrungen.

Dieses Stück hängt von der Länge des Nadelabschnitts und von der Länge der Kanäle 82 bzw. der Hälften 70, 72 ab, die mit je einer Stirnseite auf der Haut anliegen müssen. Nach dem Einstich kann durch eine Reduzierung der Kraft auf die Griffstükke 78, 80, 86 bzw. den Bügel 88 das Auseinanderspreizen der Hälften 70, 72 bewirkt werden. In dieser Stellung lassen sich die in Figuren 1 bis 3 dargestellten Vorrichtungen von der Injektionsnadel entfernen. Die Injektionsnadel kann anschließend durch eine entsprechende Handhabung der Injektionsspritze tiefer in das Gewebe eingeführt werden.

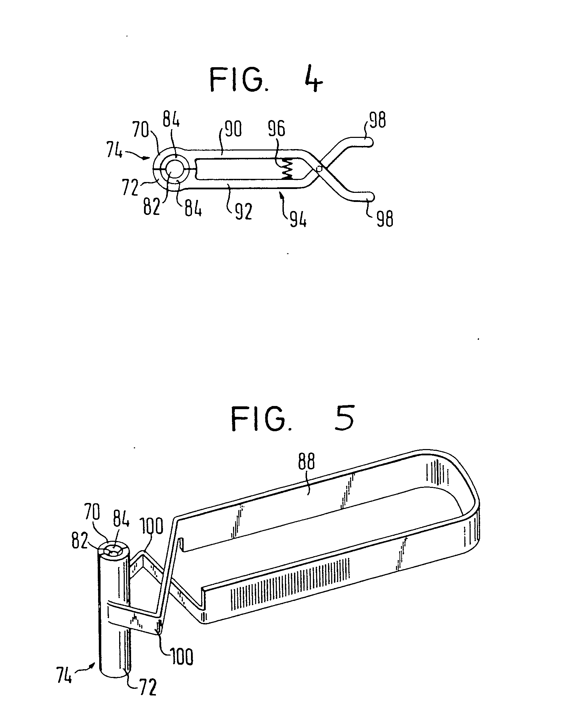

Während bei den in Figuren 1 bis 3 dargestellten Vorrichtungen die Hälften 70, 72 ohne äußere Krafteinwirkung auseinandergespreizt sind, ist in Figur 4 eine Vorrichtung gezeigt, bei der die Hälften 70, 72 ohne äußere Krafteinwirkung gegeneinander gedrückt werden. Die Hälften 70, 72 sind je mit einer Backe 90 bzw. 92 einer Zange 94 verbunden. Die beiden Backen 90, 92 werden durch eine Feder 96 in Schließrichtung vorgespannt. Durch manuelle Krafteinwirkung auf die Griffstücke 98 der Zange 94 werden die Backen 90, 92 und damit die Hälften 70, 72 auseinandergespreizt. Die in Figur 4 dargestellte Vorrichtung umgibt den Nadelabschnitt ohne manuelle Hilfe. Die Handhabung ist deshalb in vielen Fällen einfacher. Der gleiche Zweck wird einfacher mit einer Ausführungsform gemäß Fig. 5 erzielt. Hierbei sind die Schenkel 88 einer pinzettenförmigen Zange so gekreuzt, daß die Hälften 70, 72 je von Abkröpfungen 100 gehalten sind.

Prinzipiell weisen die beschriebenen Vorrichtungen je einen Führungskörper von festgelegter Länge mit Nadelführungskanal und im Verhältnis zum Hohlnadelquerschnitt erheblich größerer Auflagefläche auf, der an der Einstichstelle auf das Gewebe aufgesetzt wird. Die Hohlnadel, die im Führungskanal leicht verschiebbar ist, wird mit aufgesetzter Injektionsspritze in den Führungskörper eingeschoben, um zunächst mit der Nadelspitze auf der Gewebeoberfläche zu ruhen. Durch Krafteinwirkung auf die Injektionsspritze wird dann die Injektionsnadel um die vorgegebene Länge in das Gewebe eingeführt.

**Patentansprüche**

1. Injektionsspritze mit einem Führungskörper (74), welcher die Injektionsnadel konzentrisch umgibt und einen Kanal als Gleitbahn für die Injektionsnadel enthält, mit einem an den Körper des Patienten anzulegenden Ende, in dem eine Injektionsnadel zwischen einer eingezogenen Lage und einer Lage, bei der sie um einen bestimmten Betrag aus dem Führungskörper (74) hervorsteht, verschiebbar ist, dadurch gekennzeichnet, daß der Führungskörper (74) zwei mit einem Träger (76) verbundene, symmetrisch zueinander angeordnete gleiche Hälften (70, 72) mit hälftig ausgebildeten Kanälen (82) und Ausnehmungen (84), welche einen Anschlag für eine Injektionsnadelhalterung oder für eine mit dieser verbundenen Hülse bilden, enthält, daß die Hälften (70, 72) mittels des Trägers (76) aneinander legbar und auseinander spreizbar sind, und daß die den Ausnehmungen (84) abgewandte Stirnseite des Führungskörpers (74) eine Kanalöffnung aufweist, die von einer Auflagefläche umgeben ist, deren Flächeninhalt ein vielfaches der Querschnittsfläche der Kanalöffnung ist.

2. Vorrichtung nach Anspruch 1, dadurch gekennzeichnet, daß die beiden Hälften (70, 72) je an einem Ende von Griffstücken (78, 80) einer Zange befestigt sind, deren Griffstücke (78, 80) je an einem Ende aneinander befestigt und durch Federkraft auseinanderspreizbar sind.

3. Vorrichtung nach Anspruch 1, dadurch gekennzeichnet, daß die Zange einen federnden, U-förmig gebogenen Bügel (88) enthält, dessen Enden mit den Hälften (70, 72) verbunden sind.

4. Vorrichtung nach Anspruch 1, dadurch gekennzeichnet, daß die Zange zwei Backen (90, 92) aufweist, an deren Enden die Hälften (70, 72) befestigt sind und die von einer in Schlußstellung der Zange (54) wirkenden Federspannung beanschlagt sind (Fig. 4).

5. Vorrichtung nach Anspruch 1, dadurch gekennzeichnet, daß die Zange zwei sich kreuzende Bügel (88) aufweist (Fig. 5).

6. Vorrichtung nach einem der vorausgehenden Ansprüche, dadurch gekennzeichnet, daß der Führungskörper (74) aus einem physiologisch inaktiven Kunststoff besteht.

7. Vorrichtung nach Anspruch 6, dadurch gekennzeichnet, daß der Kunststoff Polyäthylen oder Polytetrafluoräthylen ist.

**Claims**

1. Hypodermic syringe with a guide body (74) concentrically surrounding the hypodermic needle and containing a channel as a slideway for the hypodermic needle, with an end which is to be applied to the body of the patient, in which a hypodermic needle is movable between a retracted position and a position in which it protrudes from the guide body (74) by a certain amount, characterised in that the guide body (74) comprises two identical halves (70, 72) which are connected to a support (76) and arranged symmetrically to each other, with channels (82) and recesses (84) which are formed in halves and which form a stop for a hypodermic needle holder or for a sleeve connected thereto, that the halves (70, 72) can be placed against each other and spread apart from each other by means of the support (76), and that the end of the guide body (74) which is remote from the recesses (84) has a channel opening which is surrounded by a contact surface, the surface area of which is a multiple of the area of cross-section of the channel opening.

2. Device according to Claim 1, characterised in that the two halves (70, 72) are each attached to one end of grips (78, 80) of pincers, the grips (78, 80) of which are each attached to one end and can be spread apart by spring force.

3. Device according to Claim 1, characterised in that the pincers comprise a resilient stirrup (88) which is curved in a U-shape and the ends of which are connected to the halves (70, 72).

4. Device according to Claim 1, characterised in that the pincers have two jaws (90, 92), to the ends of which are attached the halves (70, 72) and which are acted on by spring tension which acts in the closed position of the pincers (54) (Fig. 4).

5. Device according to Claim 1, characterised in that the pincers have two stirrups (88) which cross each other (Fig. 5).

6. Device according to one of the preceding Claims, characterised in that the guide body (74) consists of a physiologically inactive plastic.

7. Device according to Claim 6, characterised in that the plastic is polyethylene or polytetrafluorethylene.

**Revendications**

1. Seringue d'injection avec un corps de guidage (74) qui entoure de manière concentrique l'aiguille d'injection et qui comporte un canal en guise de guide pour l'aiguille d'injection, avec une extrémité à appliquer sur le corps du patient et dans lequel peut coulisser une aiguille d'injection, entre une position de retrait et une position dans laquelle elle sort d'une valeur déterminée du corps de guidage (74), caractérisé en ce que le corps de guidage (74) comporte deux moitiés identiques (70, 72) disposées symétriquement l'une par rapport à l'autre, reliées à un support (76), et comportant des demi-canaux (82) et des demi-évidements (84) qui constituent une butée pour un porte-aiguille d'injection ou un fourreau relié à celle-ci, en ce que les moitiés (70, 72) peuvent être appliquées l'une contre l'autre ou écartées l'une de l'autre, au moyen du support (76), et en ce que la face frontale du corps de guidage (74), opposée aux évidements (84), présente une ouverture de canal entourée par une surface d'appui dont la surface effective est un multiple de la surface de la section de l'ouverture de canal.

2. Dispositif selon la revendication 1, caractérisé en ce que les deux moitiés (70, 72) sont fixées chacune à une extrémité des poignées (78, 80) d'une pince, pince dont les poignées (78, 80) sont fixées l'une à l'autre au niveau d'une de leurs extrémités, et pouvant être écartées l'une de l'autre sous l'effet de la force d'un ressort.

3. Dispositif selon la revendication 1, caractérisé en ce que la pince comporte un étrier élastique (88) plié en forme de «U», dont les extrémités sont reliées aux moitiés (70, 72).

4. Dispositif selon la revendication 1, caractérisé en ce que la pince comporte deux mors (90, 92) aux extrémités desquels sont fixées les moitiés (70, 72), et qui sont sollicités par la tension d'un ressort agissant dans le sens de la fermeture de la pince (Fig. 4).

5. Dispositif selon la revendication 1, caractérisé en ce que la pince comporte deux étriers (88) qui se croisent (Fig. 5).

6. Dispositif selon l'une des revendications précédentes, caractérisé en ce que le corps de guidage (74) se compose d'une matière plastique physiologiquement inerte.

7. Dispositif selon la revendication (6), caractérisé en ce que la matière plastique est du polyéthylène ou du polytétrafluoréthylène.

FIG. 1

FIG.3

FIG. 2

7

## FIG. 4

## FIG. 5